# EUROPÄISCHE PATENTSCHRIFT

(11) **EP 1 537 821 B1**
(45) Veröffentlichungstag und Bekanntmachung des Hinweises auf die Patenterteilung: **25.11.2009**
(21) Anmeldenummer: 04090479.9
(22) Anmeldetag: 02.12.2004
(51) Int. Cl.: A47L 23/20

(54) **Vorrichtung zum Trocknen von Schuhen, Handschuhen, Kleidungsstücken oder dergleichen**
Device for drying shoes, gloves, garments or the like
Appareil pour sécher des chaussures, gants, vêtements ou similaires

(30) Priorität: 05.12.2003 DE 10358042
(43) Veröffentlichungstag der Anmeldung: 08.06.2005
(73) Patentinhaber: Therm-IC Products GmbH, 8200 Gleisdorf (AT)
(72) Erfinder: Macher, David, 8570 Voitsberg (AT)
(74) Vertreter: Pfenning, Meinig & Partner GbR

(56) Entgegenhaltungen:
- EP-A- 1 121 874
- EP-A- 1 253 848
- GB-A- 452 256
- US-A- 3 203 112
- US-A- 5 289 642
- US-A1- 2003 150 126
- US-A1- 2004 068 888
- PATENT ABSTRACTS OF JAPAN Bd. 1996, Nr. 10, 31. Oktober 1996 (1996-10-31) -& JP 08 141055 A (FUJITA SANAI), 4. Juni 1996 (1996-06-04)

## Beschreibung

Die Erfindung betrifft eine Vorrichtung zum Trocknen von Schuhen, Handschuhen, Kleidungsstücken oder dergleichen nach dem Oberbegriff des Hauptanspruchs.

Eine derartige Vorrichtung ist aus der EP 1 253 848 B1 bekannt. Diese Vorrichtung weist ein in einem Gehäuse aufgenommenes Gebläse auf, das einen Luftstrom erzeugt, der zu zwei gegenüberliegende Luftaustrittsöffnungen geführt wird, wobei in den Luftaustrittsöffnungen formschlüssig hohe Stutzen mit daran angesetzten Rohren eingesetzt sind. An den Rohrenden sind Luftaustrittsdüsen angeordnet aus denen gewärmte Luft austritt. Die Rohre lassen sich beispielsweise in Schuhe einstecken, die durch den warmen Luftstrom getrocknet oder aufgeheizt werden können.

Aus der japanischen Patentanmeldung 08 141 055 A ist ein Trockner beispielsweise für Schuhe bekannt, der ein Gehäuse mit einem Gebläse und einer Heizvorrichtung sowie einer Luftaustrittsdüse umfasst. Weiterhin weist er einen Ozongenerator mit alkalischer Keramik auf, der Ozon zur Desinfektion und Geruchsverbesserung erzeugt.

Aus der US 2003/0150126 ist ein Fön bekannt, der eine Vorrichtung zur Geruchsverbesserung als Zubehör aufweist. Die Vorrichtung weist einen auf den Fön aufsteckbares längliches Gehäuse auf, das eine Duftsubstanz aufnimmt. Das Gehäuse und der Fön weisen eine Öffnung auf, die durch einen mit dem Einschalter verbundenen Schieber abdeckbar oder zu öffnen ist.

US 2003/0150126 offenbart den Oberbegriff des Anspruchs 1.

Der Erfindung liegt die Aufgabe zugrunde, eine Vorrichtung zum Trocknen von Schuhen, Handschuhen, Kleidungsstücken oder dergleichen zu schaffen, die zur Geruchsverbesserung eine Vorrichtung zur Geruchsverbesserung in einer einfachen Konstruktion aufweist.

Diese Aufgabe wird erfindungsgemäß durch die kennzeichnenden Merkmale des Hauptanspruchs gelöst.

Durch die in den Unteransprüchen angegebenen Maßnahmen sind vorteilhafte Weiterbildungen und Verbesserungen möglich.

Zur Geruchsverbesserung und -neutralisierung, insbesondere bei einer gerade frisch hergestellten Vorrichtung, die üblicherweise ein Gehäuse aus Kunststoff aufweist, ist ein mit ätherischen Ölen getränkter saugfähiger Träger vorgesehen, der in dem Rohr angeordnet ist und der zur Neutralisierung des Kunststoffgeruchs Duft ausströmt, der von dem Luftstrom mitgenommen wird. Da der z.B. als Platte ausgebildete Träger in einem Hohlraum aufgenommen ist, der bei Betrieb des Gebläses geöffnet ist, wird die Lebensdauer der Duftplatte verlängert. Durch Herausziehen des Teleskoprohrs wird eine Öffnung im Hohlraum freigesetzt, die im zusammengeschobenen Zustand abgedeckt ist.

Dadurch, dass eine Einrichtung zur Erzeugung von Ozon mit einer oder mehreren Leuchtdioden, die im UV-Wellenbereich abstrahlt, vorgesehen ist, über deren Strahlung Ozon erzeugbar ist, wird eine Möglichkeit zur Erzeugung von Ozon, das sowohl zur Geruchsneutralisierung als auch zur Desinfektion dient, zur Verfügung gestellt, die wenig Platzbedarf benötigt und somit die Vorrichtung nicht vergrößert.

In vorteilhafter Weise ist die mindestens eine UV-Leuchtdiode auf einer Schaltungsplatine bzw. -folie angeordnet, die innerhalb des Gehäuses festgelegt ist und die weitere elektrische und/oder elektronische Bauelemente, wie eine oder mehrere Leuchtdioden zur Funktionsanzeige, Zeitsteuerelemente oder Spannungsverringerungselemente oder dergleichen tragen kann.

Ein Ausführungsbeispiel der Erfindung ist in der Zeichnung dargestellt und wird in der nachfolgenden Beschreibung näher erläutert. Es zeigen:
- Fig. 1: eine Seitenansicht auf die erfindungsgemäße Vorrichtung,
- Fig. 2: eine Aufsicht auf das offene Gehäuse eines nicht die Erfindung darstellenden Beispiel,
- Fig. 3: eine vergrößerte Darstellung des Gehäuseinneren nach Fig. 2 mit Leuchtdioden,
- Fig. 4: einen Schnitt durch ein Teleskoprohr mit Hohlraum im zusammengesteckten und auseinandergezogenen Zustand mit Vergrößerung, und
- Fig. 5: ein schematischer Schnitt durch einen Hohlraum mit Abdeckung, nach einem erläuternden, nicht die Erfindung darstellenden Beispiel.

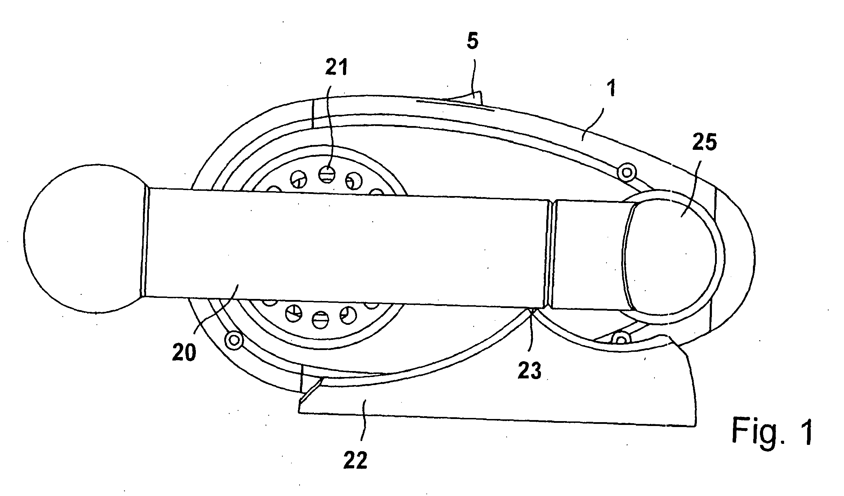

Die in Fig. 1 dargestellte Vorrichtung zum Trocknen und Aufheizen von Schuhen, Handschuhen oder Kleidungsstücken weist ein Gehäuse 1 und zwei an das Gehäuse angesetzte Rohre 20 (nur eines ist zu sehen), die Armen gleich neben dem Gehäuse 1 angeordnet sind, auf. Eine solche Vorrichtung ist in der EP 1 253 848 B1 offenbart. Im Gehäuse sind ein elektrisch betreibbares Gebläse 4 sowie elektrische Bauelemente zur Ansteuerung des Gebläses 4 aufgenommen, wobei die elektrische Versorgung über ein nicht dargestelltes Kabel von einem Netz und/oder einer Batterie und/oder einem aufladbaren Akkumulator geliefert werden kann. Zum Ein- und Ausschalten des Gebläses 4 ist ein Schalter 5 in einer schmalen Seitenwand des Gehäuses 1 eingelassen. Im Bereich des Gebläses 4 ist das Gehäuse 1 mit Ansaugöffnungen 21 versehen, durch die Luft von außen angesaugt wird. Ein Aufstellelement 22 mit Anschlägen 23 für die Rohre 20 kann integraler Bestandteil des Gehäuses 1 sein, oder lösbar mit diesem verbunden sein.

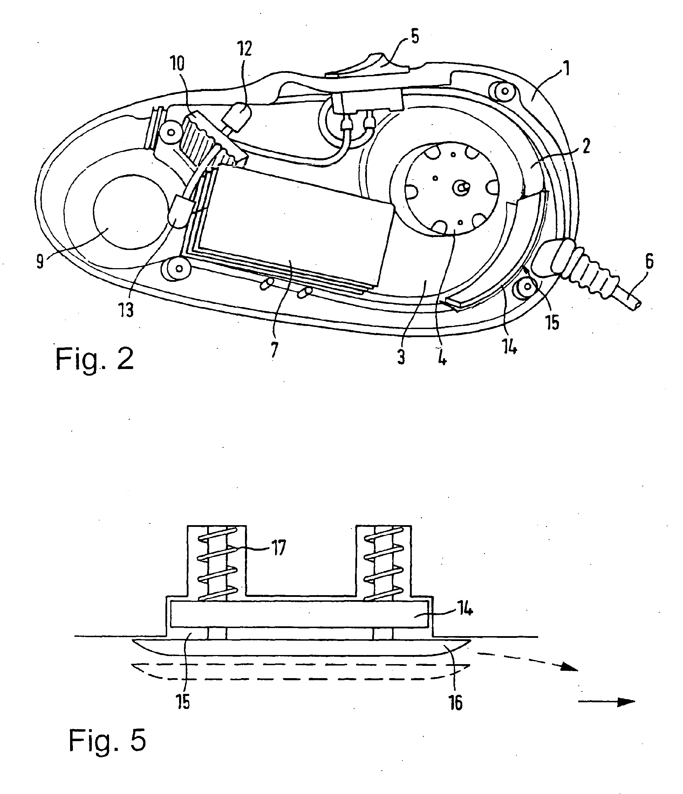

In Fig. 2 nach einem erläuternden, nicht die Erfindung darstellenden Beispiel, ist ein offenes Gehäuseteil dargestellt, wobei das Gehäuse 1 aus Kunststoff besteht und im Inneren des Gehäuses Stege oder Wände 2 eingeformt und ausgebildet sind, die einen Luftkanal 3 definieren. Innerhalb der Kanalwände 2 ist das Gebläse 4 zur Erzeugung eines Luftstroms angeordnet, das über den Schalter 5 mit einer Spannungsversorgungsleitung 6 verbunden ist.

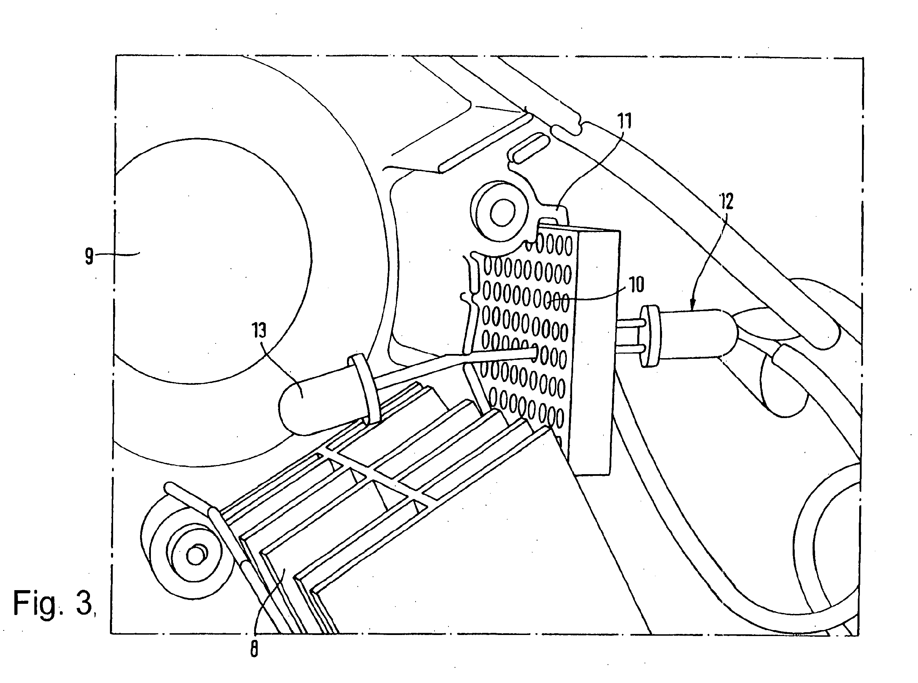

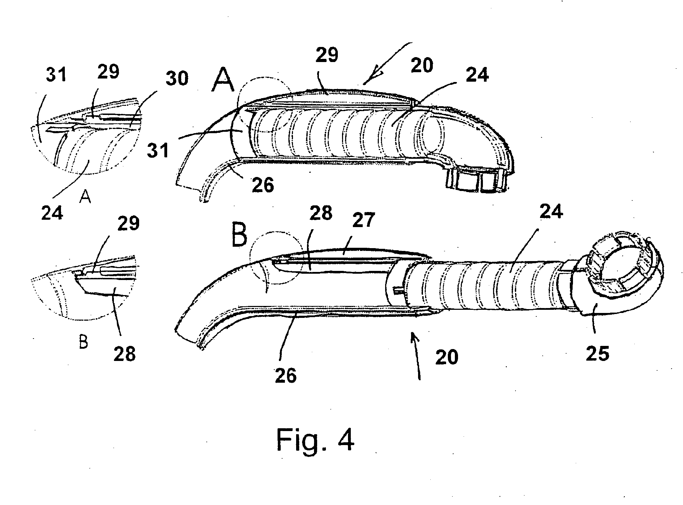

In dem Luftkanal 3 ist stromabwärts ein mit Rippen 8 versehener Kühlblock 7 (siehe auch Fig. 3), in bzw. an dem nicht dargestellte Heizelemente gelagert sind, die den durchströmenden Luftstrom vom Gebläse 4 aufheizen. Weiter stromabwärts ist im Gehäuse 1 eine Luftaustrittsöffnung 9 vorgesehen, wobei eine weitere Luftaustrittsöffnung in dem das dargestellte Gehäuseteil abdeckenden Gehäuseteil ausgeformt ist. In dieser Luftaustrittsöffnung 9 ist ein Rohrstutzen formschlüssig und drehbar eingesetzt, in dem wiederum das Teleskoprohr 20 aufgenommen ist (s. Fig. 4), dessen offenes Ende eine Luftdüse bildet oder mit speziellen Düsen versehen ist.

Nach einem erläuternden, nicht die Erfindung darstellenden Beispiel, ist in der Wandung 2 des Luftkanals 3 ein Hohlraum 15 eingearbeitet, wie er näher in Fig. 5 dargestellt ist. In diesem Hohlraum 15 befindet sich ein mit ätherischen Ölen getränkter saugfähiger Träger, z.B. Kartonstreifen 14. Dabei wird vorzugsweise der Duft der ätherischen Öle über eine kleine Fläche des Kartonstreifens emittiert, damit er diese Funktion länger beibehält. Dieser Duftstreifen 14 dient insbesondere zum Neutralisieren des Geruchs des Kunststoffes, aus dem das Gehäuse 1 hergestellt ist. Dieser Geruch ist besonders bei neu gespritzten Kunststoffteilen vorhanden, und er verliert sich, wenn die Kunststoffteile länger in Gebrauch sind. Daher ist der mit ätherischen Ölen versehene Kartonstreifen 14, dessen Duft sich gleichfalls mit sehr langem Gebrauch verliert, bei einer neuen Vorrichtung besonders nützlich. Die ätherischen Öle werden so ausgewählt, dass ihr Duft den Kunststoffgeruch besonders gut neutralisiert.

Um jedoch die Lebensdauer des Duftstreifens zu verlängern, ist nach einem erläuternden, nicht die Erfindung darstellenden Beispiel, der Hohlraum 15 mit einer Abdeckung 16 versehen, die im Betrieb des Gebläses geöffnet wird, ansonsten jedoch geschlossen ist. Dazu ist die Abdeckung durch zwei Rückstellfedern 17 in dem geschlossenen Zustand vorgespannt. Bei Vorhandensein eines Luftstroms wird durch den erzeugten Unterdruck die Abdeckung abgehoben und das Duftmittel wird an den Luftstrom abgegeben.

Es sind jedoch andere Schließ- bzw. Öffnungsvorrichtungen für die Abdeckung bzw. für ein den Hohlraum abschließendes Verschlusselement möglich, wobei eine erfindungsgemäße Möglichkeit in Fig. 4 dargestellt ist.

Fig. 4 zeigt oben ein Teleskoprohr 20 im eingesteckten Zustand und darunter ein solches im ausgezogenen Zustand, wobei mit A und B vergrößerte Ausschnitte bezeichnet sind. Das Teleskoprohr 20 besteht aus zwei Rohrteilen, einem feststehenden Rohrteil 24, das über den Rohrstutzen 25 formschlüssig in der Luftaustrittsöffnung 9 aufgenommen ist, und einem auf dem feststehenden Rohrteil 24 verschiebbaren Rohrteil 26. Im Wandbereich des verschiebbaren Rohrteils 26 ist eine Kammer 27 eingearbeitet, die zu dem den Luftstrom führenden Rohrinneren durch eine Wand 28 abgeschlossen ist, die eine Verbindungsöffnung 29 aufweist. In dieser Kammer 27 ist der Duftträger angeordnet.

Wie aus Fig. 4 oben zu erkennen ist, ist zwischen der Wand des verschieblichen Rohrteils 26 bzw. der Wand 28 und dem feststehenden Rohrteil 24 im zusammengesteckten Zustand ein Ringspalt 30 ausgebildet, der durch eine auf dem feststehenden Rohrteil 24 sitzende Manschette 31 nach außen, d.h. zum offenen Ende des Rohrteils 26 abgeschlossen ist.

Der in der Kammer 27 angeordnete Duftträger strömt einen Duft durch die Verbindungsöffnung 29 aus, der im zusammengefalteten Zustand des Teleskoprohres 20 im Ringspalt 30 "gefangen" ist. Wenn das Rohr 20 für den Gebrauch auseinandergezogen wird, wird der Duft mit dem durch das Gebläse 4 erzeugten Luftstrom mitgenommen und außerdem durch die dann nach außen geöffnete Verbindungsöffnung 29 gesandt. Der den Duft einschließende Hohlraum, der abgeschlossen ist, wenn das Gerät nicht in Betrieb ist, wird somit aus der Kammer 27 und dem Ringspalt 30 gebildet.

Wie in Fig. 3 dargestellt ist, ist eine Schaltungsplatine 10 in Führungen 11 im Gehäuseinneren eingesetzt, in der mindestens eine Leuchtdiode 12 zur Funktionsanzeige und eine UV-Leuchtdiode 13 verankert sind. Die Leuchtdiode 12 zur Funktionsanzeige liegt hinter einem Fenster in dem Gehäuse 1, durch das das Licht der Leuchtdiode hindurchstrahlt, wenn die Vorrichtung eingeschaltet ist. Die UV-Leuchtdiode 13 liegt stromabwärts vom Kühlblock vor der Luftaustrittsöffnung 9. Selbstverständlich können mehrere UV-Leuchtdioden 13 im Luftstrom angeordnet und auf der Schaltungsplatine 10 eingesetzt sein. Ebenso kann die Schaltungsplatine weitere elektronische und/oder elektrische Bauelemente zur Steuerung der Funktionsweise der Vorrichtung enthalten.

Wenn das Gebläse 4 der Vorrichtung eingeschaltet wird, werden gleichfalls die Leuchtdiode 12 zur Funktionsanzeige, die im Kühlkörper 7 vorhandenen Heizelemente und die mindestens eine UV-Leuchtdiode 13 eingeschaltet. Die von der UV-Leuchtdiode 13 abgestrahlte UV-Strahlung erzeugt zusammen mit dem in dem Luftstrom vorhandenen Sauerstoff Ozon, das mit den Luftstrom zu dem zu trocknenden oder aufzuwärmenden Gegenstand transportiert wird und zum Desinfizieren und Geruchsneutralisieren dient.

## Patentansprüche

1. Vorrichtung zum Trocknen von Schuhen, Handschuhen, Kleidungsstücken oder dergleichen mit einem einen Luftstrom erzeugenden Gebläse (4), das in einem Gehäuse (1) aufgenommen ist, wobei das Gehäuse (1) mindestens eine Luftaustrittsöffnung (9) aufweist, die über ein Rohr (20) mit einer Luftdüse (25) in Verbindung steht, und wobei eine Einrichtung zur Geruchsverbesserung vorgesehen ist,
**dadurch gekennzeichnet, dass** die Einrichtung zur Geruchsverbesserung als ein mit ätherischen Ölen getränkter saugfähiger Träger (14) ausgebildet ist, der einen Duft, insbesondere zum Neutralisieren des Geruchs des aus Kunststoff bestehenden Gehäuses (1) abgibt und der in einem verschließbaren Hohlraum (27) aufgenommen ist, der bei Betrieb des Gebläses (4) geöffnet ist, wobei der Hohlraum (27) in dem Rohr (20) vorgesehen ist und das Rohr (20) als aus mehreren Rohrteilen (24,26) bestehendes Teleskoprohr (20) ausgebildet ist, wobei die Öffnung (29) des Hohlraumes (27) im zusammengesteckten Zustand durch ein Rohrteil geschlossen ist und im ausgezogenen Zustand freigelegt ist.

2. Vorrichtung nach Anspruch 1, **dadurch gekennzeichnet, dass** eine Einrichtung zur Erzeugung von Ozon zum Desinfizieren im Wirkbereich des Luftstroms vorgesehen ist.

3. Vorrichtung nach Anspruch 2, **dadurch gekennzeichnet, dass** die Einrichtung zur Erzeugung von Ozon mindestens eine UV-Leuchtdiode (13) aufweist, über deren UV-Strahlung zusammen mit dem Sauerstoff im Luftstrom das Ozon erzeugbar ist.

4. Vorrichtung nach Anspruch 3, **dadurch gekennzeichnet, dass** innerhalb des Gehäuses (1) Wände (2) zur Bildung eines Luftkanals (3) vom Gebläse (4) zur Luftaustrittsöffnung (9) vorgesehen sind, wobei in dem Luftkanal (3) ein Kühlblock (7) zur Aufnahme von Heizelementen für ein Aufheizen des durch den Kühlblock (7) geleiteten Luftstroms angeordnet ist und dass die mindestens eine UV-Leuchtdiode (13) zwischen Kühlblock (7) und Luftaustrittsöffnung (9) angeordnet ist.

5. Vorrichtung nach einem der Ansprüche 1 bis 4, **dadurch gekennzeichnet, dass** die mindestens eine UV-Leuchtdiode (13) auf einer Schaltungsplatine (10) angeordnet ist, die über einen Schalter (5) mit einer Spannungsversorgung verbindbar ist.

6. Vorrichtung nach einem der Ansprüche 1 bis 5, **dadurch gekennzeichnet, dass** in die Luftaustrittsöffnung (9) formschlüssig ein drehbarer Rohrstutzen mit sich daran anschließendem Rohr eingesetzt ist.

7. Vorrichtung nach Anspruch 6, **dadurch gekennzeichnet, dass** zwei gegenüberliegende Luftaustrittsöffnungen (9) mit entsprechenden Rohrstutzen und Rohren vorgesehen sind.

8. Vorrichtung nach einem der Ansprüche 5 bis 7, **dadurch gekennzeichnet, dass** die Schaltungsplatine (10) weitere elektrische und/oder elektronische Bauelemente trägt.

## Claims

1. Device for drying shoes, gloves, garments or the like, having a fan (4) which produces an airflow and is received in a housing (1), the housing (1) having at least one air outlet opening (9) which is connected via a tube (20) to an air nozzle (25), and a mechanism for odour improvement being provided,
***characterised in that***
the mechanism for odour improvement is configured as an absorbent carrier (14) which is saturated with ether oils, said carrier emitting a scent, in particular for neutralising the odour of the housing (1) which comprises plastic material and is received in a closable cavity (27) which is opened during operation of the fan (4), the cavity (27) being provided in the tube (20) and the tube (20) being configured as a telescopic tube (20) which comprises a plurality of tube parts (24, 26), the opening (29) of the cavity (27) being closed in the retracted state by a tube part and being uncovered in the extended state.

2. Device according to claim 1, **characterised in that** a mechanism for producing ozone is provided for disinfection in the active region of the airflow.

3. Device according to claim 2, **characterised in that** the mechanism for producing ozone has at least one UV light diode (13), via the UV radiation of which, together with the oxygen in the airflow, ozone can be produced.

4. Device according to claim 3, **characterised in that**, within the housing (1), walls (2) are provided in order to form an air channel (3) from the fan (4) to the air outlet opening (9), a cooling block (7) being disposed in the air channel (3) for receiving heating elements for heating the airflow which is conducted through the cooling block (7) and **in that** the at least one UV light diode (13) is disposed between cooling block (7) and air outlet opening (9).

5. Device according to one of the claims 1 to 4, **characterised in that** the at least one UV light diode (13) is disposed on a circuit board (10) which can be connected via a switch (5) to a voltage supply.

6. Device according to one of the claims 1 to 5, **characterised in that** a rotatable tube connection piece with a tube connected thereto is inserted in a form fit in the air outlet opening (9).

7. Device according to claim 6, **characterised in that** two oppositely situated air outlet openings (9) with corresponding tube connection pieces and tubes are provided.

8. Device according to one of the claims 5 to 7, **characterised in that** the circuit board (10) carries further electrical and/or electronic components.

## Revendications

1. Dispositif pour sécher des chaussures, des gants, des vêtements ou similaires avec une soufflerie (4) générant un courant d'air, qui est logée dans un boîtier (1), dans lequel le boîtier (1) présente au moins une ouverture de sortie d'air (9), qui est en communication, via un tube (20), avec une tuyère à air (25) et dans lequel il est prévu un dispositif pour améliorer l'odeur,
**caractérisé en ce que** le dispositif d'amélioration d'odeur se présente sous la forme d'un support absorbant (14) imprégné d'huiles essentielles, qui dégage un parfum, en particulier pour neutraliser l'odeur du boîtier (1) constitué d'un matériau synthétique et qui est logé dans une cavité verrouillable (27), qui est ouverte lors du fonctionnement de la soufflerie (4), ladite cavité (27) étant prévue dans le tube (20) et le tube (20) se présentant sous la forme d'un tube télescopique (20) constitué de plusieurs parties de tube (24, 26), l'ouverture (29) de la cavité (27) étant fermée à l'état rentré par une partie de tube et dégagée à l'état déployé.

2. Dispositif selon la revendication 1, **caractérisé en ce qu'**il est prévu un dispositif pour générer de l'ozone afin de désinfecter dans la zone d'action du courant d'air.

3. Dispositif selon la revendication 2, **caractérisé en ce que** le dispositif pour générer de l'ozone présente au moins une diode électroluminescente UV (13), dont le rayonnement UV conjointement avec l'oxygène présent dans le courant d'air peut générer de l'ozone.

4. Dispositif selon la revendication 3, **caractérisé en ce qu'**il est prévu dans le boîtier (1) des parois (2) pour former un canal à air (3) de la soufflerie (4) à l'ouverture de sortie d'air (9), dans lequel on agence, dans le canal à air (3), un bloc de refroidissement (7) destiné à recevoir des éléments chauffants pour un chauffage du courant d'air envoyé à travers le bloc de refroidissement (7) et **en ce que** l'on agence la au moins une diode électroluminescente UV (13) entre le bloc de refroidissement (7) et l'ouverture de sortie d'air (9).

5. Dispositif selon l'une quelconque des revendications 1 à 4, **caractérisé en ce que** la au moins une diode électroluminescente UV (13) est aménagée sur une platine de commutation (10) qui peut être connectée via un commutateur (5) à une source de tension.

6. Dispositif selon l'une quelconque des revendications 1 à 5, **caractérisé en ce que** l'on installe par complémentarité de forme dans l'ouverture de sortie d'air (9) un raccord tubulaire rotatif avec le tube s'y raccordant.

7. Dispositif selon la revendication 6, **caractérisé en ce que** l'on prévoit deux ouvertures de sortie d'air opposées (9) avec des raccords tubulaires et des tubes correspondants.

8. Dispositif selon l'une quelconque des revendications 5 à 7, **caractérisé en ce que** la platine de commutation (10) porte d'autres composants électriques et/ou électroniques.
